# EUROPÄISCHE PATENTSCHRIFT

(11) **EP 0 442 340 B1**
(45) Veröffentlichungstag und Bekanntmachung des Hinweises auf die Patenterteilung: **22.02.1995**
(21) Anmeldenummer: 91101437.1
(22) Anmeldetag: 04.02.1991
(51) Int. Cl.: C07F 1/02, C07C 45/00, C07C 51/00, C07F 7/08

(54) **Verfahren zur Lithiierung von 1,3-Bis(trifluormethyl)benzol**
Process for the preparation of an organolithium compound starting from 1,3-bis(trifluoromethyl)-benzene
Procédé pour la préparation d'un composé organolithié à partir de 1,3-bis(trifluorométhyl)-benzène

(30) Priorität: 13.02.1990 CH 463/90
(43) Veröffentlichungstag der Anmeldung: 21.08.1991
(73) Patentinhaber: F. HOFFMANN-LA ROCHE AG, 4002 Basel (CH)
(72) Erfinder: Masciadri, Raffaello, Dr., CH-4055 Basel (CH)
(74) Vertreter: Notegen, Eric-André

(56) Entgegenhaltungen:
- FR-A- 1 499 563
- GB-A- 1 080 167
- US-A- 3 751 491
- J. CHEM. SOC. (C), 1971, Seiten 3305-3308; D.E. GROCOCK et al.: "Metallation of
- 1,3-bistrifluoromethylbenzene and NN-dimethyl-3,5-bistrifluoromethylaniline"
- JOURNAL OF ORGANOMETALLIC CHEMISTRY, Band 67, Nr. 3, 19. März 1975, Seiten 321-325, Elsevier Sequoia S.A., Lausanne, CH; P. AEBERLI et al.: "The metalation of1,3-bis(trifluoromethyl)benzene by n-butyllithium"
- BULLETIN OF THE CHEMICAL SOCIETY OF JAPAN, Band 61, Nr. 5, Mai 1988, Seiten1625-1631, The Chemical Society of Japan; K. KODAIRA et al.: "Preparation offluorine-containing phenylacetylenes by the method of introduction of theethynyl group using 1,1-dichloro-2,2-difluoroethene"
- JOURNAL OF ORGANOMETALLIC CHEMISTRY, Band 303, Nr. 3, 15. April 1986, Seiten429-435, Elsevier Sequoia S.A., Lausanne, CH; H.A. BRUNE et al.: "Synthesen voncis-(Phenyl)[4-(trimethylsilyl)phenyl]bis(triphenylphosphan)platin(II)-Verbindungen mit substituenten unterschiedlichen Charakters in den Phenyl-Ringen"

## Beschreibung

Die vorliegende Erfindung betrifft ein Verfahren zur Lithiierung von 1,3-Bis(trifluormethyl)benzol in einem für diesen Zweck geeigneten Lösungsmittel, das dadurch gekennzeichnet ist, dass man die Lithiierung mit dem Lithiumsalz eines Amins der allgemeinen Formel

R¹R²NH I

worin R¹ und R² je eine sekundäre oder tertiäre niedere Alkylgruppe oder eine gegebenenfalls durch niederes Alkyl substituierte niedere Cycloalkylgruppe oder R¹ und R² zusammen eine C₆₋₁₄-Alkylengruppe, in welcher die beiden mit dem Stickstoffatom verknüpften Kohlenstoffatome sekundär oder tertiär und durch 2 bis 4 Kohlenstoffatome voneinander getrennt sind, darstellen,
durchführt.

In einem weiteren Aspekt betrifft die vorliegende Erfindung ein Verfahren zur Herstellung von Verbindungen der allgemeinen Formel 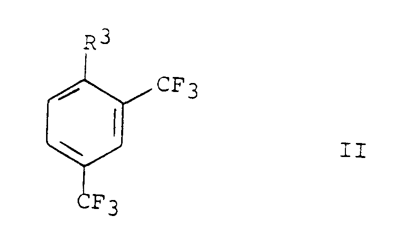
worin R³ den Rest eines für die Substitution von lithiierten Benzolderivaten geeigneten Elektrophils bedeutet,
das dadurch gekennzeichnet ist, dass man die erfindungsgemäss erhaltene Lösung des lithiierten 1,3-Bis(trifluormethyl)benzols mit einem Elektrophil zur Umsetzung bringt.

Ein weiterer Gegenstand der vorliegenden Erfindung ist die Verwendung von Lithiumsalzen von Aminen der obigen Formel I bei der Lithiierung von 1,3-Bis(trifluormethyl)benzol.

Die erfindungsgemäss erhältlichen Verbindungen der Formel II sind wertvolle Zwischenprodukte, welche für die Herstellung der verschiedensten Produkte verwendet werden können. Beispielsweise können sie für die Herstellung von pharmazeutischen Wirkstoffen verwendet werden, welche als Strukturmerkmal eine 2,4-Bis(trifluormethyl)phenylgruppe besitzen, z.B. für die Herstellung von 4-[(Z)-2,4-Bis(trifluormethyl)styryl]-4,8-dimethyl -2,3-dioxabicyclo[3.3.1]nonan-7-on, das in der Europäischen Patentpublikation Nr. 311 955 beschrieben ist und zur Verhütung oder Bekämpfung der Malaria verwendet werden kann.

Die Lithiierung von 1,3-Bis(trifluormethyl)benzol und die Umsetzung der erhaltenen Lösung mit Elektrophilen sind an sich bekannte Reaktionen, vgl. K. Kodaira et al. in Bull. Chem. Soc. 61, 1625-1631 (1988) (Dokument A), J.P. Coleman et al. in J. Chem. Soc. Perkin I 1973, 1903 ff. (Dokument B), P. Aeberli et al. in J. Organomet. Chem. 67, 321-325 (1974) (Dokument C) und in US-A-3.751.491 - W.J. Houlihan (Dokument D). In diesen Dokumenten wird die Lithiierung von 1,3-Bis(trifluor-methyl)benzol mit n-Butyllitihium und die Umsetzung der erhaltenen Lösungen mit Elektrophilen, nämlich mit elementarem Brom bzw. festem Kohlendioxid, beschrieben. Als Produkte werden Gemische von Bis(trifluormethyl)-brombenzolen bzw. -benzoesäuren erhalten, welche im wesentlichen aus dem entsprechenden 2,4-Isomeren und aus dem entsprechenden 2,6-Isomeren bestehen, d.h. die Lithiierung und die anschliessende Reaktion mit den Elektrophilen findet hauptsächlich in der 2- und in der 4-Stellung des 1,3-Bis(trifluormethyl)benzols statt. Bezüglich der Zusammensetzung der gemäss diesen Dokumenten erhaltenen Gemische wird auf die nachfolgende Tabelle I verwiesen.

**Tabelle I**

| Dokument | Elektrophil | R³ | Anteil der Isomeren in % | | |
|---|---|---|---|---|---|
| | | | 2,4-Isomer | 2,6-Isomer | andere Isomere |
| Dokument A | Br₂ | Br | 47,5 | 45,1 | 7,4 |
| Dokument A | " | " | 47,3 | 42,4 | 10,3 |
| Dokument B | CO₂ fest | -COOH | 56,3 | 35,0 | 8,7 |
| Dokument C | " | " | 60,0 | 40,0 | - |
| Dokument C | " | " | 62,0 | 38,0 | - |
| Dokument C | " | " | 59,0 | 41,0 | - |
| Dokument D | " | " | 65,0 | 35,0 | - |

Wie dieser Tabelle entnommen werden kann, variiert das Verhältnis 2,4-Isomer/2,6-Isomer in den erhaltenen Produkten zwischen etwa 1:1 und 3:2 zugunsten des 2,4-Isomeren.

Es wurde nun überraschenderweise gefunden, dass der Anteil an 2,4-Isomer im erhaltenen Produkt dramatisch erhöht werden kann, wenn man für die Lithiierung das Lithiumsalz eines Amins der obigen Formel I verwendet, vgl. Tabelle II im Anschluss an den experimentellen Teil. Durch die erfindungsgemässen Verfahren erhält man Produkte, in welchen das Verhältnis 2,4-Isomer (d.h. Verbindung der Formel II) zu 2,6-Isomer von 4:1 bis über 100:1 variiert. Durch die dramatische Verschiebung des Verhältnisses zugunsten der 2,4-Isomeren wird deren Abtrennung aus den Isomeren-Gemischen und deren Reinigung natürlich enorm erleichtert.

Die erfindungsgemässe Lithiierung wird vorzugsweise in einem niederen, offenkettigen oder cyclischen Aether oder in einem Gemisch davon mit einem offenkettigen oder cyclischen niederen Kohlenwasserstoff durchgeführt. Die Reaktionstemperatur liegt vorzugsweise in einem Bereich von etwa -80°C bis etwa Raumtemperatur, vorzugsweise jedoch unterhalb 0°C. Als Lithiierungsmittel verwendet man vorzugsweise das Lithiumsalz von 2,2,6,6-Tetramethylpiperidin, Diisopropylamin, t-Butylisopropylamin, Di-t-butylamin, t-Butylcyclohexylamin oder Dicyclohexylamin, insbesondere das Lithiumsalz von 2,2,6,6-Tetramethylpiperidin.

Zur Herstellung von Verbindungen der Formel II kann man entweder die erfindungsgemäss erhaltene Lösung des lithiierten 1,3-Bis(trifluormethyl)benzols zum Elektrophil geben oder das Elektrophil, vorzugsweise so rasch wie möglich, zur erfindungsgemäss erhaltenen Lösung des lithiierten 1,3-Bis(trifluormethyl)benzols geben.

In einer besonders bevorzugten Ausführungsform verwendet man als Elektrophil elementares Halogen, festes Kohlendioxid, N,N-Dimethylformamid oder Methyljodid und isoliert als Produkt eine Verbindung der Formel II, worin R³ Brom, Carboxy, Formyl bzw. Methyl bedeutet. In einer ganz besonders bevorzugten Ausführungsform verwendet man als Elektrophil festes Kohlendioxid oder N,N-Dimethylformamid und isoliert als Produkt 2,4-Bis(trifluormethyl)benzoesäure bzw. 2,4-Bis(trifluormethyl)benzaldehyd.

Der in der vorliegenden Beschreibung verwendete Ausdruck "nieder" bezeichnet Reste und Verbindungen mit höchstens 7, vorzugsweise höchstens 4 Kohlenstoffatomen. Der Ausdruck "Alkyl" bezeichnet geradkettige oder verzweigte, gesättigte Kohlenwasserstoffreste, wie Methyl, Aethyl, Propyl, Isopropyl und t-Butyl. Der Ausdruck "Cycloalkyl" bezeichnet cyclische, gesättigte Kohlenwasserstoffreste, wie Cyclopentyl und Cyclohexyl.

Der Ausdruck "Elektrophil" bezeichnet Verbindungen, welche in der Lage sind mit lithiierten Benzolderivaten im Sinne einer Addition oder Substitution zu reagieren. Geeignete Elektrophile sind beispielsweise die Halogene Brom und Jod, nicht enolisierbare, d.h. aromatische oder α,ß-ungesättigte, Aldehyde und Ketone, N,N-Di(niedere Alkyl)amide und cyclische N-Formyl- und N-(niedere Alkanoyl)amine, Kohlendioxid und niedere Alkylhalogenide.

Die nachfolgenden Beispiele dienen der näheren Erläuterung der vorliegenden Erfindung. Sie sollen deren Umfang jedoch in keine Weise beschränken. Sämtliche Temperaturen sind in Celsiusgraden angegeben.

### Beispiel 1

### Lithiierung von 1,3-Bis(trifluormethyl)benzol

Man löst 27,2 ml (0,16 Mol) 2,2,6,6-Tetramethylpiperidin in 400 ml Tetrahydrofuran und kühlt diese Lösung unter einem Argonstrom auf -40° ab. Dann gibt man über einen Zeitraum von 10 Minuten 100 ml einer 1,6M Lösung von n-Butyllithium in Hexan bei -40° dazu. Man erwärmt die gelbliche Lösung mit einem Wasserbad kurz auf 0°, kühlt dann auf -75° ab und gibt über einen Zeitraum von 15 Minuten 20,2 ml (0,13 Mol) 1,3-Bis(trifluormethyl)benzol bei -75° tropfenweise dazu. Man rührt die erhaltene violette Lösung noch während einer Stunde bei -75°.

### Umsetzung von lithiiertem 1,3-Bis(trifluormethyl)benzol mit Elektrophilen

Zur obigen violetten Lösung gibt man bei -75° mindestens 0,16 Mol des Elektrophils so rasch wie möglich dazu. In Folge der exothermen Reaktion erwärmt sich dabei die Lösung um 10-30°. Man lässt das Reaktionsgemisch dann auf 0° erwärmen und giesst es dann langsam unter Rühren zu 500 ml einer kalten 3M Salzsäurelösung. Man verdünnt mit 300 ml Hexan und trennt die wässrige Phase ab. Die organische Phase wird mit 500 ml kalter 3M Salzsäurelösung extrahiert, zweimal mit je 500 ml gesättigter Kochsalzlösung neutral gewaschen, über Natriumsulfat getrocknet und filtriert, worauf man das Lösungsmittel abdestilliert. Man reinigt das erhaltene Rohprodukt durch Kristallisation oder Destillation über eine geeignete Kolonne.

Mittels dieses Verfahrens wurden die nachfolgend beschriebenen Verbindungen erhalten:
a) 2,4-Bis(trifluormethyl)benzoesäure

| | |
|---|---|
| Elektrophil: | festes Kohlendioxid im Ueberschuss. |
| Ausbeute: | 80%. |
| Smp. | 104°. |
| ¹H-NMR (CDCl₃): | 7,95 (d,1H,J=8Hz); 8,07 (s,1H); 8,12 (d,1H,J=8Hz) ppm. |
| MS (EI) m/e: | 258 (M⁺), 241 (M⁺-OH), 213, 194, 163, 144. |

b) 1-Methyl-2,4-bis(trifluormethyl)benzol

| | |
|---|---|
| Elektrophil: | Methyljodid. |
| Ausbeute: | 60%. |
| Sdp. | 140°. |
| ¹H-NMR (CDCl₃): | 2,56 (s,3H); 7,43 (d,1H,J=8Hz); 7,69 (d,1H,J=8Hz); 7,86 (s,1H) ppm. |
| MS (EI) m/e: | 228 (M⁺), 209 (M⁺-F), 159 (M⁺-CF₃). |

c) 1-(Trimethylsilyl)-2,4-bis(trifluormethyl)benzol

| | |
|---|---|
| Elektrophil: | Trimethylchlorsilan. |
| Ausbeute: | 65%. |
| Sdp. | 75°/1,47 kPa. |
| ¹H-NMR (CDCl₃): | 0,373; 0,377; 0,382 (3xs,9H); 7,74 (d,1H,J=7,8Hz); 7,86 (d,1H,J=7,8Hz); 7,92 (s,1H) ppm. |
| MS (EI) m/e: | 271 (M⁺-CH₃), 267 (M⁺-F), 231, 151. |

d) 2,4-Bis(trifluormethyl)benzaldehyd

| | |
|---|---|
| Elektrophil: | N,N-Dimethylformamid. |
| Ausbeute: | 30%. |
| Sdp. | 65°/1,47 kPa. |
| ¹H-NMR (CDCl₃): | 7,99 (d,1H,J=8,1Hz); 8,05 (s,1H); 8,27 (d,1H,J=8,1Hz); 10,44 (m,1H) ppm. |
| MS (EI) m/e: | 242 (M⁺), 241 (M⁺-H), 223 (M⁺-F), 222 (M⁺-HF), 213 (M⁺-CHO), 195, 194, 164, 163, 145, 144. |

e) 1-Brom-2,4-bis(trifluormethyl)benzol

| | |
|---|---|
| Elektrophil: | Brom. |
| Ausbeute: | 43%. |
| Sdp. | 95°/10 kPa. |
| ¹H-NMR (CDCl₃): | 7,66 (dd,1H,J₁=8,4Hz,J₂=2Hz); 7,88 (d,1H,J₁=8,4Hz); 7,94 (d,1H,J₂=2Hz) ppm. |
| MS (EI) m/e: | 294 (M⁺), 292 (M⁺), 273 (M⁺-F), 213 (M⁺-Br). |

### Beispiel 2 (mit Temperatur-Variation)

Man kühlt eine Lösung von y Mol 2,2,6,6-Tetramethylpiperidin in 400 ml wasserfreiem Tetrahydrofuran unter einem Argonstrom auf t ° ab und tropft bei dieser Temperatur innert 30 Minuten 100·y/0,16 ml einer 1,6M Lösung von n-Butyllithium in Hexan dazu. Anschliessend tropft man bei t ° innert 15 Minuten 20,2 ml (0,13 Mol) 1,3-Bis(trifluormethyl)benzol dazu, rührt die erhaltene weinrote Lösung noch während x Minuten bei t ° und lässt dann bei dieser Temperatur 20 ml (0,26 Mol) N,N-Dimethylformamid aus einem Tropftrichter schnell einfliessen. Infolge der exothermen Reaktion steigt die Innentemperatur t dabei um etwa 15° an. Die erhaltene dunkelrote Lösung tropft man dann langsam und unter Rühren und Kühlen zu 500 ml kalter 3M Salzsäure (stark exotherm). Man verdünnt die erhaltene Emulsion mit 300 ml Hexan, trennt die wässrige Phase ab, extrahiert die organische Phase mit 500 ml kalter 3M Salzsäure, wäscht zweimal mit je 250 ml gesättigter Kochsalzlösung neutral, trocknet über Natriumsulfat und destilliert die organischen Lösungsmittel bei 40° Badtemperatur/20 kPa weitgehend ab. Den Rückstand destilliert man über eine 20 cm lange Kolonne, wobei man zunächst bei 50° Badtemperatur/20 kPa restliche Lösungsmittel entfernt und dann die Badtemperatur auf 80° und das Vakuum auf 1,4 kPa erhöht. Während die Innentemperatur ansteigt nimmt man einen Vorlauf von etwa 1 g und destilliert dann den erhaltenen 2,4-Bis(trifluormethyl)benzaldehyd bei 56°/1,4 kPa als farblose Flüssigkeit über.

### Ergebnisse:

| Reaktionstemperatur t | Reaktionszeit x | Menge Base y | Ausbeute | Reinheit (GC) |
|---|---|---|---|---|
| -30° | 20 Min. | 0,16 Mol (27,2 ml) | 70% | 98% |
| -20° | 15 Min. | 0,16 Mol (27,2 ml) | 70% | 98% |
| -10° | 3 Min. | 0,16 Mol (27,2 ml) | 70% | 98% |
| -10° | 5 Min. | 0,13 Mol (22,1 ml) | 70% | 96% |

| | |
|---|---|
| ¹H-NMR (CDCl₃): | 7,99 (d,1H,J=8,1Hz); 8,05 (s,1H); 8,27 (d,1H,J=8,1Hz); 10,44 (m,1H) ppm. |
| MS, Spitzen bei m/e: | 242 (M⁺), 241 (M⁺-H), 223 (M⁺-F), 222 (M⁺-HF), 213 (M⁺-CHO), 195, 194, 164, 163, 145, 144. |

### Beispiel 3

a) Man kühlt eine Lösung von 68 ml (0,4 Mol) 2,2,6,6-Tetramethylpiperidin in 1 l Tetrahydrofuran unter Argon auf -10° ab und tropft bei dieser Temperatur unter Rühren 250 ml einer 1,6M Lösung von n-Butyllithium in Hexan dazu. Anschliessend tropft man bei -10° innert 5 Minuten 62 ml (0,4 Mol) 1,3-Bis(trifluormethyl)benzol dazu. Man rührt die resultierende weinrote Lösung noch während 5 Minuten bei -10° und lässt dann 62 ml (0,8 Mol) N,N-Dimethylformamid schnell einfliessen. Infolge der exothermen Reaktion steigt die Innentemperatur um 15° an. Die erhaltene dunkelbraune Lösung gibt man dann langsam und unter leichtem Argondruck zu 1,2 l gerührte, eisgekühlte 1M Salzsäure. Infolge der stark exothermen Reaktion steigt die Innentemperatur trotz stetem Kühlen auf 10°. Man verdünnt die resultierende Emulsion mit 750 ml Hexan, trennt die wässrige Phase (1,5 l) ab und bewahrt sie zur Rückgewinnung von 2,2,6,6-Tetramethylpiperidin auf. Man extrahiert die organische Phase (1,9 l) zweimal mit je 1 l Wasser, trocknet über Natriumsulfat, filtriert und destilliert die organischen Lösungsmittel bei 40° unter Vakuum (20 kPa) ab. Den Rückstand (etwa 100 ml) destilliert man über eine 20 cm lange Kolonne, zunächst bei 50° Badtemperatur unter einem Vakuum von 20 kPa zur Entfernung der restlichen Lösungsmittel, dann erhöht man die Badtemperatur auf 80° und das Vakuum auf 1,4 kPa. Man nimmt einen Vor lauf bis die konstante Innentemperatur von 57° erreicht ist und destilliert dann den Rest als eine Fraktion bei 57°/1,4 kPa über. Man erhält 68 g (70%) 2,4-Bis(trifluormethyl)benzaldehyd mit einer Reinheit (GC) von 96-98%.
b) Rückgewinnung von 2,2,6,6-Tetramethylpiperidin
   Man extrahiert die oben aufbewahrte saure wässrige Phase (1,5 l) einmal mit 1 l Diäthyläther, kühlt sie auf 10° ab, gibt unter Rühren und Kühlen 200 ml rohe 28-proz. Natronlauge dazu und sättigt mit Kochsalz. Man extrahiert die erhaltene basische Lösung einmal mit 1,5 l Diäthyläther, trocknet die organische Phase über Natriumsulfat, filtriert und destilliert den Aether bei 40° im Vakuum (70 kPa) ab. Den Rückstand (etwa 200 ml) destilliert man bei Normaldruck über eine 20 cm lange Kolonne, wobei die restlichen Lösungsmittel (40-110°), ein Vorlauf (110-150°) und schliesslich 53 g (94%) 2,2,6,6-Tetramethylpiperidin (155°) mit einer Reinheit (GC) von 94-99% überdestillieren.

### Beispiel 4

Man kühlt eine Lösung von 100 ml (0,21 Mol) Lithiumdiisopropylamid in Tetrahydrofuran unter Argon auf -70° ab und tropft bei dieser Temperatur unter Rühren 31 ml (0.2 Mol) 1,3-Bis(trifluormethyl)benzol dazu. Man rührt die resultierende dunkelrote, dickflüssige Suspension noch während 30 Minuten bei -70° und lässt dann 31 ml (0,4 Mol) N,N-Dimethylformamid schnell einfliessen. Infolge der exothermen Reaktion steigt die Innentemperatur trotz anhaltender Kühlung auf -20° an. Die erhaltene violette Lösung wird dann wie in Beispiel 3a) beschrieben weiterverarbeitet. Man erhält 2,4-Bis(trifluormethyl)benzaldehyd.

**Tabelle II**

| Gaschromatographische Analyse der Rohprodukte, enthaltend die jeweilige Verbindung der Formel II: | | | | | |
|---|---|---|---|---|---|
| Beispiel | Elektrophil | R³ | Anteil der Isomeren in % | | |
| | | | 2,4-Isomer | 2,6-Isomer | andere Isomere |
| 1e) | Br₂ | Br | 79,7 | 20,3 | - |
| 1a) | CO₂ fest | -COOH | >90 | <10 | - |
| 1b) | CH₃-J | -CH₃ | 92,2 | 6,5 | 1,3 |
| 1c | ClSi(CH₃)₃ | -Si(CH₃)₃ | 90,8 | 8,1 | 1,1 |
| 1d) | DMF | -CHO | 93,1 | 6,9 | - |
| 2 (-30° | " | " | 98, 1 | 0,7 | 1,2 |
| 2 (-20°) | " | " | 98,6 | 0,5 | 0,9 |
| 2 (-10°) | " | " | 98,1 | 0,8 | 1,1 |
| 3a) | " | " | 98,4 | 0,8 | 0,8 |
| 4 | " | " | 34,0 | <1,0 | <1,0 |
| DMF = N,N-Dimethylformamid | | | | | |

## Patentansprüche

1. Verfahren zur Lithiierung von 1,3-Bis(trifluormethyl)benzol in einem für diesen Zweck geeigneten Lösungsmittel, dadurch gekennzeichnet, dass man die Lithiierung mit dem Lithiumsalz eines Amins der allgemeinen Formel
R¹R²NH I
worin R¹ und R² je eine sekundäre oder tertiäre niedere Alkylgruppe oder eine gegebenenfalls durch niederes Alkyl substituierte niedere Cycloalkylgruppe oder R¹ und R² zusammen eine C₆₋₁₄-Alkylengruppe, in welcher die beiden mit dem Stickstoffatom verknüpften Kohlenstoffatome sekundär oder tertiär und durch 2 bis 4 Kohlenstoffatome voneinander getrennt sind, darstellen,
durchführt.

2. Verfahren nach Anspruch 1, dadurch gekennzeichnet, dass man als Lösungsmittel einen niederen, offenkettigen oder cyclischen Aether oder ein Gemisch davon mit einem offenkettigen oder cyclischen niederen Kohlenwasserstoff verwendet.

3. Verfahren nach Anspruch 1 oder 2, dadurch gekennzeichnet, dass man das Lithiumsalz von 2,2,6,6-Tetramethylpiperidin verwendet.

4. Verfahren zur Herstellung von Verbindungen der allgemeinen Formel 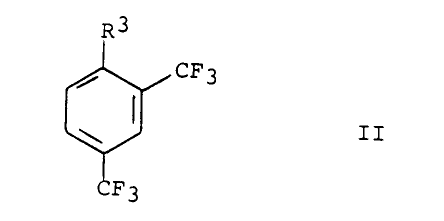 worin R³ den Rest eines für die Substitution von lithiierten Benzolderivaten geeigneten Elektrophils bedeutet,
dadurch gekennzeichnet, dass man die nach einem der Ansprüche 1 bis 3 erhaltene Lösung des lithiierten 1,3-Bis(trifluormethyl)benzols mit einem Elektrophil zur Umsetzung bringt.

5. Verfahren nach Anspruch 4, dadurch gekennzeichnet, dass man das Elektrophil, vorzugsweise so rasch wie möglich, zur Lösung des lithiierten 1,3-Bis(trifluormethyl)benzols gibt.

6. Verfahren nach Anspruch 4, dadurch gekennzeichnet, dass man die Lösung des lithiierten 1,3-Bis(trifluormethyl)benzols zum Elektrophil gibt.

7. Verfahren nach einem der Ansprüche 4 bis 6, dadurch gekennzeichnet, dass man als Elektrophil elementares Brom, festes Kohlendioxid, N,N-Dimethylformamid oder Methyljodid verwendet und als Produkt eine Verbindung der Formel II isoliert, worin R³ Brom, Carboxy, Formyl bzw. Methyl bedeutet.

8. Verfahren nach Anspruch 7, dadurch gekennzeichnet, dass man als Elektrophil festes Kohlendioxid verwendet und als Produkt 2,4-Bis(trifluormethyl)benzoesäure isoliert.

9. Verfahren nach Anspruch 7, dadurch gekennzeichnet, dass man als Elektrophil N,N-Dimethylformamid verwendet und als Produkt 2,4-Bis(trifluormethyl)benzaldehyd isoliert.

10. Verwendung von Lithiumsalzen von Aminen der in Anspruch 1 definierten Formel I bei der Lithiierung von 1,3-Bis(trifluormethyl)benzol.

11. Verwendung des Lithiumsalzes von 2,2,6,6-Tetramethylpiperidin bei der Lithiierung von 1,3-Bis(trifluormethyl)benzol.

## Claims

1. A process for the lithiation of 1 ,3-bi(trifluoromethyl)benzene in a solvent which is suitable for this purpose, characterized in that the lithiation is carried out with the lithium salt of an amine of the general formula
R¹R²NH I
wherein R¹ and R² each signify a secondary or tertiary lower alkyl group or a lower cycloalkyl group which is optionally substituted by lower alkyl or R¹ and R² together signify a C₆₋₁₄-alkylene group in which the two carbon atoms linked with the nitrogen atom are secondary or tertiary and are separated from each other by 2 to 4 carbon atoms.

2. A process according to claim 1, characterized in that a lower open-chain or cyclic ether or a mixture thereof with an open-chain or cyclic lower hydrocarbon is used as the solvent.

3. A process according to claim 1 or 2, characterized in that the lithium salt of 2,2,6,6-tetramethylpiperidine is used.

4. A process for the manufacture of compounds of the general formula 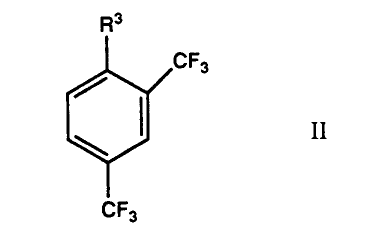 wherein R³ signifies the residue of an electrophile which is suitable for the substitution of lithiated benzene derivatives,
characterized by reacting the solution of the lithiated 1,3-bis(trifluoromethyl)benzene obtained according to any one of claims 1 to 3 with an electrophile.

5. A process according to claim 4, characterized in that the electrophile is added, preferably as rapidly as possible, to the solution of the lithiated 1,3-bis(trifluoromethyl)benzene.

6. A process according to claim 4, characterized in that the solution of the lithiated 1,3-bis(trifluoromethyl)benzene is added to the electrophile.

7. A process according to any one of claims 4 to 6, characterized in that elementary bromine, solid carbon dioxide, N,N-dimethylformamide or methyl iodide is used as the electrophile and a compound of formula II in which R³ signifies bromine, carboxy, formyl or, respectively, methyl is isolated as the product.

8. A process according to claim 7, characterized in that solid carbon dioxide is used as the electrophile and 2,4-bis(trifluoromethyl)benzoic acid is isolated as the product.

9. A process according to claim 7, characterized in that N,N-dimethylformamide is used as the electrophile and 2,4-bis(trifluoromethyl)benzaldehyde is isolated as the product.

10. The use of lithium salts of amines of formula I defined in claim 1 in the lithiation of 1,3-bis(trifluoromethyl)benzene.

11. The use of the lithium salt of 2,2,6,6,-tetramethylpiperidine in the lithiation of 1 ,3-bis(trifluoromethyl)benzene.

## Revendications

1. Procédé pour l'addition de lithium au 1,3-bis(trifluorométhyl)benzène dans un solvant approprié à cette fin, caractérisé en ce que l'on effectue l'addition de lithium à l'aide du sel de lithium d'une amine de formule générale
R¹R²NH I
dans laquelle R¹ et R² représentent chacun un groupe alkyle inférieur secondaire ou tertiaire, ou un groupe cycloalkyle inférieur éventuellement substitué par un groupe alkyle inférieur, ou R¹ et R² forment ensemble un groupe alkylène en C₆-C₁₄ dans lequel les deux atomes de carbone liés à l'atome d'azote sont secondaires ou tertiaires et sont séparés l'un de l'autre par 2 à 4 atomes de carbone.

2. Procédé selon la revendication 1, caractérisé en ce que l'on utilise comme solvant un éther inférieur à chaîne ouverte ou cyclique, ou un mélange de tels éthers avec un hydrocarbure inférieur à chaîne ouverte ou cyclique.

3. Procédé selon la revendication 1 ou 2, caractérisé en ce que l'on utilise le sel de lithium de la 2,2,6,6-tétraméthylpipéridine.

4. Procédé pour la préparation de composés de formule générale 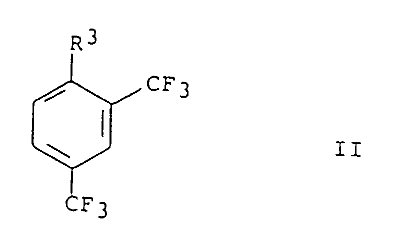 dans laquelle R³ représente le reste d'un composé électrophile approprié à la substitution de dérivés benzéniques lithiés,
caractérisé en ce que l'on met en réaction avec un composé électrophile la solution du 1,3-bis(trifluorométhyl)benzène lithié, obtenue selon l'une des revendications 1 à 3.

5. Procédé selon la revendication 4, caractérisé en ce que l'on ajoute, de préférence aussi rapidement que possible, le composé électrophile à la solution du 1,3-bis(trifluorométhyl)benzène lithié.

6. Procédé selon la revendication 4, caractérisé en ce que l'on ajoute la solution du 1,3-bis(trifluorométhyl)benzène lithié au composé électrophile.

7. Procédé selon l'une des revendications 4 à 6, caraactérisé en ce que, comme composé électrophile, on utilise le brome élémentaire, le dioxyde de carbone solide, le N,N-diméthylformamide ou l'iodure de méthyle, et on isole en tant que produit un composé de formule II dans lequel R³ représente l'atome de brome ou le groupe carboxy, formyle ou méthyle.

8. Procédé selon la revendication 7, caractérisé en ce que l'on utilise comme composé électrophile le dioxyde de carbone solide et on isole en tant que produit de l'acide 2,4-bis(trifluorométhyl)benzoïque.

9. Procédé selon la revendication 7, caractérisé en ce que l'on utilise comme composé électrophile le N,N-diméthylformamide et on isole en tant que produit du 2,4-bis(trifluorométhyl)benzaldéhyde.

10. Utilisation de sels lithiés d'amines de formule I définie dans la revendication 1, dans l'addition de lithium au 1,3-bis(trifluorométhyl)benzène.

11. Utilisation du sel lithié de la 2,2,6,6-tétraméthylpipéridine dans l'addition de lithium au 1,3-bis-(trifluorométhyl)benzène.
